# EUROPEAN PATENT SPECIFICATION

(11) **EP 0 307 097 B1**
(45) Date of publication and mention of the grant of the patent: **09.06.1993**
(21) Application number: 88307610.1
(22) Date of filing: 17.08.1988
(51) Int. Cl.: A61K 37/02, A61K 37/66

(54) **Water-insolubilized cytokines**
Wasserunlösliche Cytokine
Cytokines rendues insolubles dans l'eau

(30) Priority: 08.09.1987 JP 224381/87
(43) Date of publication of application: 15.03.1989
(73) Proprietor: Takeda Chemical Industries, Ltd., Chuo-ku, Osaka (JP)
(72) Inventor: Mikura, Yasushi, Osaka 565 (JP); Shimizu, Hisayoshi, Osaka 567 (JP); Toguchi, Hajime, Hyogo 663 (JP); Sato, Jun, Hyogo 662 (JP)
(74) Representative: Laredo, Jack Joseph

(56) References cited:
- EP-A- 0 138 216
- EP-A- 0 140 255
- EP-A- 0 230 647
- EP-A- 0 251 631
- EP-A- 0 281 299
- WO-A-83/00150

## Description

This invention relates to water-insolubilized forms of cytokines which are useful as sustained release injections, for instance, to compositions for injection which comprises said water-insolubilized forms of cytokines and to a method for producing said water-insolubilized forms of cytokines.

As known preparations for attaining prolongation of the duration of drug activity, there may be mentioned the aqueous insulin zinc suspension for injection and aqueous protamine zinc insulin suspension for injection (Japanese Pharmacopeia, 11th edition).

They are preparations of water-insoluble forms of insulin, which is low in water solubility.

Methods for producing sustained - release preparations of cytokines are known in the art.

EP-A-0230647 describes a general method for producing a sustained release formulation which comprises collagen and/or gelatin as a carrier while EPA 0140255 discloses a sustained-release injection which contains an active ingredient and a specific biodegradable carrier.

However, in developing physiologically active substances as drug, various problems are encountered which arise from the characteristic properties of the substances. For instance, interleukin-2 (IL-2), which is a cytokine and is strongly expected to serve as a drug, shows a short serum concentration half-life of about 0.6 hours following intravenous administration and of about 1.3 hours following intramuscular administration. For causing more efficient expression of its therapeutic effects, development is required of preparations endowed with the properties of sustained release and target-orientedness.

Accordingly, based on the idea that rendering IL-2 insoluble in water might result in sustained release thereof because a certain period of time would be needed for the insolubilization product to release water-solubilized IL-2, the present inventors continued their studies and, as a result, found that when inorganic salts, such as sodium chloride, calcium chloride zinc chloride and phosphates, low-molecular-weight organic acids and salts thereof, such as ammonium acetate and sodium citrate, and amino acids, such as glycine, are added to an aqueous solution of IL-2 to an osmotic pressure not lower than the isotonic level and the mixtures are shaken, water-insolubilized forms of IL-2 are produced, with the physiological activities of IL-2 being reduced to approximately half of the original, however.

Under the circumstances mentioned above, the present inventors made intensive investigations in an attempt to obtain physiological activity-retaining and water-insolubilized forms of cytokines and found that such physiological activity-retaining and water-insolubilized forms of cytokines can be obtained only when cytokines are treated with a biocompatible protein or a biocompatible high molecular organic acid having a molecular weight of not less than 5,000 or a salt thereof in water. Further studies based on this finding have now led to completion of the present invention.

Thus the invention provides (1) physiological activity-retaining and water-insolubilized forms of cytokines, (2) compositions for injection which comprises such physiological activity-retaining and water-insolubilized forms of cytokines, (3) a method of producing physiological activity-retaining and water-insolubilized forms of cytokines, which comprises acting a physiologically adaptable protein or a physiologically adaptable high molecular organic acid having a molecular weight of not less than 5,000 or a salt thereof on a cytokine in an aqueous medium.

The cytokine includes monokines and lymphokines, among others.

As said monokines, there may be mentioned, for example, tumor necrosis factor (TNF), interleukin-1 (IL-1) and differentiation activating factor (DAF).

As said lymphokines, there may be mentioned, for example, T cell growth factor (interleukin-2 or IL-2), interferon-α, interferon-β, interferon-γ, interleukin-3, lymphotoxin (LT), macrophage migration inhibitory factor (MIF), macrophage activating factor (MAF), macrophage chemotaxis factor (MCF), B cell growth factor (BCGF), B cell differentiation factor (BCDF), neutrophil chemotaxis factor (NCF) and leukocyte migration inhibitory factor (LIF).

Hereinafter, interleukin-2 is referred to as IL-2 for short, interferon-α as IFN-α, interferon-β as IFN-β, and interferon-γ as IFN-γ.

The IL-2 may be a substance substantially identical in activity to IL-2, namely a substance capable of maintaining T cells in subculturing while maintaining the functions of T cells. More specifically, it may be the polypeptide (I) (human IL-2) having the amino acid sequence shown in Japanese Patent Application No. 78799/86 which corresponds to EP Publication No. 176299 (in Fig. 1 thereof) or a fragment thereof comprising a partial amino acid sequence necessary for the expression of the biological or immunological activities of IL-2. As such fragment, there may be mentioned, among others, a fragment which is otherwise identical to the polypeptide (I) but lacks the amino-terminal one amino acid (EP Publication No. 91539) or the amino-terminal four amino acids (Japanese Patent Application Laid-open No. 126088/85) and a fragment of the polypeptide (I) in which several amino acids in the carboxyl terminus portion are missing. Furthermore, it may be a polypeptide derived from the polypeptide (I) having the amino acid sequence shown in Fig. 1 in the above-cited publication by deletion of one or more amino acids or substitution thereof with a different amino acid or acids, for example the analog containing a serine residue in lieu of the cysteine residue in position 125 (Japanese Patent Application Laid-open No. 93093/84, which corresponds to U.S. Patent No. 4,518,584).

Furthermore, the above-mentioned IL-2-active substance may be chemically modified with a polyethylene glycol derivative, for instance (e.g. Japanese Patent Application Laid-open No. 226821/85, which corresponds to EP Publication No. 154,316).

In the practice of the invention, the use of human IL-2 having the amino acid sequence shown in Fig. 1 in Japanese Patent Application No. 78799/86 which corresponds to EP Publication No. 176,299 is particularly preferred. In that case, said human IL-2 may be used in admixture with that modification thereof which further has a methionine residue (Met) at the amino terminus (Japanese Patent Applications Laid-open No. 115528/85 and No. 78799/86 which correspond to EP Publications No. 145,390 and No. 176,299, respectively). Use may also be made of that modification which has not Met at the amino terminus but begins with alanine (Ala) (Japanese Patent Application Laid-open No. 78799/86 which corresponds to EP Publication No. 176,299).

Said IL-2 may further have a sugar chain.

The IFN-α may be any substance having IFN-α activity, namely antiviral activity. Thus it may be natural IFN-α or a genetically engineered IFN-α species. As examples of the genetically engineered IFN-α species, there may be mentioned γIFN-αA, B, C, D, E, F, G, H, I and J (Japanese Patent Application Laid-open No. 79897/82; European Patent Publication No. 43980).

The IFN-γ may be any substance which has IFN-γ activities, namely antiviral activity and immune system activating activity, and may be natural IFN-γ or a genetically engineered IFN-γ species.

As the genetically engineered IFN-γ species, there may be mentioned that species obtained by the method described in Japanese Patent Application Laid-open No. 90514/83 which corresponds to EP Publication No. 77670, that species obtained by the method described in Japanese Patent Application Laid-open No. 186995/84 which corresponds to EP Publication No. 110,044.

Those fragments of IFN-γ which lack one to several amino acids from the amino terminus and/or carboxyl terminus of IFN-γ may also be used. As such fragments, there may be mentioned those described in Japanese Patent Applications Laid-open No. 202899/85 and No. 5096/86 which correspond to EP Publications No. 146,354 and No. 166,993 respectively, among others.

The cytokines to be used in the practice of the invention which has been disclosed hereinabove are readily soluble in water and have a solubility of not less than 0.5 mg/ml, for instance.

The physiologically adaptable protein to be used in the production of the water-insolubilized forms according to the present invention includes, among others, proteins originating from the living body, such as human serum albumin, human serum globulin, fibrinogen, collagen and casein.

As the physiologically adaptable high molecular organic acid having a molecular weight of not less than 5,000, there may be mentioned, for example, alginic acid, hyaluronic acid, chondroitinsulfuric acid, pectic acid, pectinic acid, heparinic acid and polyacrylic acid as well as pectin and carrageenan mainly composed of such acids.

As the salt of said high molecular organic acid, there may be mentioned, among others, sodium salt, calcium salt, magnesium salt, zinc salt, ammonium salt, arginine salt and N-methylglucamine salt.

The action of a physiologically adaptable protein on a cytokine in an aqueous medium is effected by causing the cytokine and the physiologically adaptable protein to coexist in an aqueous medium and applying a physical stimulation thereto. The aqueous medium is, for example, composed of water such as distilled water for injection.

As said physical stimulation, there may be mentioned, for example, shaking or stirring. In carring out the above procedure, the cytokine concentration in the cytokine- and protein-containing aqueous solution is preferably not less than about 0.1 mg/ml, desirably not less than 0.5 mg/ml. The protein concentration is preferably not less than about half the cytokine concentration, desirably not less than the cytokine concentration. The upper limit to the protein concentration is preferably not more than about 500 times, more preferably not more than about 100 times, the cytokine concentration. It is recommendable that the pH of said aqueous solution should be adjusted to about 3 to 12, preferably about 4 to 11, with hydrochloric acid, sodium hydroxide, or a buffer in which an inorganic acid, such as phosphoric acid, an organic acid, such as acetic acid or citric acid, or an amino acid, such as glycine, is used, for instance. For promoting insolubilization in water, for instance, there may be added, in addition to said protein, an inorganic salt, such as sodium chloride, calcium chloride or zinc chloride, an organic acid or a salt thereof, such as ammonium acetate or sodium citrate, or an amino acid, such as glycine, to said aqueous solution.

Said shaking is effected by shaking a container containig the pH-adjusted, cytokine- and protein-containing aqueous solution on a vortex mixer or a shaker. The intensity and duration of shaking which are required may vary depending on the composition of the aqueous solution, the liquid volume and other factors. In principle, however, the shaking should preferably be vigorous and prolonged to a certain extent so that the yield of the water-insolubilized form can be increased. More specifically, the shaking period preferably being from 5 seconds to 30 minutes, more preferably 30 seconds to 10 minutes.

Said stirring is effected by stirring the pH-adjusted, cytokine- and protein-containing aqueous solution using a propeller stirrer, a magnetic stirrer or a homogenizer. The intensity and duration of stirring which are necessary may vary depending on the composition of the aqueous solution, the liquid volume and other factors. In principle, however, the stirring should preferably be intense and prolonged to a certain extent so that the yield of the water insolubilized form can be increased. More specifically, the stirring period is preferably from 5 seconds to 30 minutes, more preferably from 30 seconds to 10 minutes.

The physiologically adaptable high molecular organic acid having a molecular weight of not less than 5,000 or a salt thereof is caused to act on the cytokine by adding said high molecular organic acid or its salt to an aqueous solution of the cytokine.

The cytokine concentration and the concentration of said high molecular organic acid in the cytokine-and high molecular organic acid-containing aqueous solution and the preferred pH conditions are as mentioned above for the case of adding the physiologically adaptable protein.

An inorganic acid salt, such as sodium chloride, calcium chloride, magnesium chloride or zinc chloride, an organic acid or a salt thereof, such as ammonium acetate or sodium citrate, or an amino acid, such as glycine, may be added to said aqueous solution for the promotion of insolubilization in the aqueous medium. Furthermore, for causing gelation of the high molecular organic acid added, not for promoting insolubilization, calcium chloride or magnesium chloride may be added. Unlike the case where the biocompatible protein is added, shaking or stirring is not particularly needed when said high molecular organic acid is caused to act on cytokines. For the promotion of insolubilization in an aqueous medium, however, shaking or stirring may be further applied in the manner mentioned above.

The addition level of the high molecular organic acid or a salt thereof is preferably not less than about 0.5 part (by weight), more preferably about 1 to 10 parts (by weight), per part (by weight) of the cytokine.

The present water-insolubilized form of cytokine is also produced by acting a physiologically adaptable protein on a cytokine in the coexistence of a water-soluble organic solvent.

As said water-soluble organic solvent, there are mentioned acetone, methanol and ethanol.

The organic solvent is acted on the cytokine by adding the organic solvent to a solution containing the cytokine and the physiologically adaptable protein. The amount of the organic solvent to be added depends on the amount of the solution, it is preferable to use the same volume to the four times of the amount of the organic solvent relative to the volume of the solution.

If required, after adding the organic solvent to the solution, a gentle stirring may be carried out.

In the above manner, there are obtained physiological activity-retaining and water-insolubilized forms of cytokines.

The extent of the physiological activity or activities retained by the water-insolubilized forms should be not less than about 60%, more preferably not less than about 80%, as compared with the original cytokines.

The thus-obtained, physiologically active and water-insolubilized forms of cytokine can be used as sustained release injections. Said physiologically active, water-insolubilized cytokine can also be used in the form of sustained release compositions for injection as prepared by combining with various carriers.

Thus, for instance, the water-insolubilized forms according to the present invention can be used as sustained release injections since even when they are administered as they are in the form of suspensions in water or various dispersion media intramuscularly, subcutaneously or by some other route, they are solubilized at the site of administration to show sustained release characteristics. Their sustained release property can be increased by causing them to be taken up by solutions, sols, gels, jellies, matrices or microcapsules by per se known means using high molecular substances obtained from biological materials, such as albumin, collagen, fibrinogen, alginic acid, pectic acid, pectin and carrageenan, and salts thereof, or biocompatible synthetic high molecular substances, such as polylactic acid, polyglycolic acid, polylactic acid-polyglycolic acid copolymer, ethylcellulose, polydimethylsiloxane polymer, poly-ethylene, polyhydroxy methacrylate-ethylene glycol methacrylate copolymer, polyvinyl alcohol, poly-2-hydroxyethyl methacrylate, ethylene-vinyl acetate copolymer and polyoxyethylene-polyoxypropylene copolymer. Among these high molecular substances, polylactic acid, polyglycolic acid, and copolymers of these are used with particular advantage.

It is also possible to cause the water-insolubilized cytokine according to the present invention to be taken up by liposomes using phosphatidylcholine, for instance, and proceeding in the conventional manner, with suspensions of said products as aqueous phases.

Furthermore, the water-insolubilized cytokine according to the invention can be used in the form of oil-in-water type emulsions prepared in the conventional manner by suspending said products in a dried powder state in a vegetable oil, which serves as the oil phase, such as soybean oil or safflower oil, and using, as the emulsifier, natural lecithin or a surfactant such as sorbitan fatty acid ester, or in the form of water-in-oil-in-water type emulsions prepared by treating suspensions of the water-insolubilized form of cytokine according to the invention as aqueous phases, dispersing the suspensions in an oil phase and dispersing the dispersions in another aqueous phase.

The injectable compositions according to the invention may be improved in their characteristics as injections by adding, when appropriate, isotonizing agents (e.g. sodium chloride, glucose, mannitol, sorbitol), pH adjusting agents (e.g. hydrochloric acid, sodium hydroxide), stabilizers (e.g. sodium pyrosulfite, Rongalit, sodium hydrogen matasulfite), suspending agents (e.g. polyethylene glycol, polysorbate 80, glycerin, carboxymethylcellulose, polyvinyl alcohol, sodium alginate, pectin, casein, gelatin), analgesics (e.g. xylocaine hydrocloride, chlorobutanol, procaine hydrochloride) and preservatives (e.g. benzyl alcohol, phenol, methyl para-hydroxybenzoate, propyl para-hydroxybenzoate).

When the water-insolubilized cytokine according to the invention and the compositions for injection which contain said products are administered by injection, the cytokines are gradually solubilized to show sustained release property, retaining the physiological activities of the cytokines.

The water-insolubilized cytokine according to the invention thus retain the physiological activities of the cytokines. Moreover, since the insolubilization aids used are physiologically adaptable substances, the water-insolubilized forms according to the invention are very low in toxicity.

Therefore, the water-insolubilized cytokine according to the invention can be used for the same purposes as the known cytokines.

When the water-insolubilized cytokine according to the invention, which show sustained release characteristics, are administered as sustained release injections, they are administered at a dose corresponding to the number of days of the sustained release period, namely at a dose calculated by multiplying the mean daily release amount during the sustained release period, which should be equal to the known daily dose of the cytokine in question, by the number of days of the sustained release period. When necessary, said dose may be reduced to a small extent.

The water-insolubilized cytokine according to the invention satisfactorily retain the physiological activities of the cytokines. When administered to living bodies, said water-insolubilized forms are gradually solubilized, so that the physiological activities of the cytokines are exhibited for a prolonged period of time. Therefore, said water-insolubilized cytokine can be used advantageously as pharmaceutical preparations, in particular as sustained release preparations for injection.

The IL-2 used in the examples to be mentioned later herein is producible in the manner described in Japanese Patent Application Laid-open No. 115528/85 or No. 78799/86 which correspond to EP Publications No. 145,390 and No, 176,299 respectively using the transformant Escherichia coli DH1/pTF4 (IFO 14299, FERM BP-628) or Escherichia coli N4830/pTB285 (IFO 14437, FERM BP-852).

The above-mentioned transformant Escherichia coli DH1/pTF4 has been deposited since November 25, 1983 at the Institute for Fermentation, Osaka (IFO), Japan under accession number of IFO 14299. Said transformant has also been deposited at the Fermentation Research Institute, Agency of Industrial Science and Technology, Ministry of International Trade and Industry (FRI), Japan since April 6, 1984 initially under the accession number of FERM P-7578 and, after conversion under the Budapest Treaty, the transformant has been stored at FRI under the accession number of FERM BP-628.

The above-mentioned transformant Escherichia coli N4830/pTB285 has been deposited at the IFO since April 25, 1985 under the accession number of IFO 14437. This transformant has also been deposited since April 30, 1985 at the FRI initially under the accession number of FERM P-8199 and, after conversion under the Budapest Treaty, the transformant has been stored at FRI under the accession number of FERM BP-852.

In the examples given later herein, the IL-2 activity measurement was performed by the method described in Japanese Patent Application Laid-open No. 115528/85 which corresponds to EP Publication No. 145,390.

The IFN-α used in the examples to be mentioned later is producible in the manner as described in Japanese Patent Application Laid-open No. 79897/82 which corresponds to EP Publication No. 43980.

The IFN-γ used in the examples to be mentioned later is producible in the manner as described in Japanese Patent Application Laid-open No. 186995/84 which corresponds to EP Publication No. 110,044.

### Examples

The following examples illustrate the invention in further detail but are by no means limitative of the scope of the present invention.

### Example 1

A 0.5-ml portion of an IL-2 stock solution (concentration about 1 mg/ml, pH 5.0) and 5 mg of lyophilized human serum albumin were placed in each of six 10-ml glass-made pointed tubes. After dissolution of the human serum albumin, 25mM ammonium acetate buffers having different pH values were respectively added in an amount of 0.05 ml to the tubes to give six aqueous IL-2 solutions having pH values of 2.5, 3.5, 5.4, 7.9, 9.9 and 12.0, respectively. The aqueous solution in each tube was stirred vigorously for 3 minutes using a vortex mixer (Thermonics microthermomixer model TM-101.).

As a result, water-insolubilized forms were formed from the aqueous solutions having the pH values of 5.4, 7.9, and 9.9. The IL-2 contents in the water-insolubilized forms were 86% (pH 5.4), 72% (pH 7.9) and 62% (pH 9.9) based on the amount of IL-2 charged.

These water-insolubilized forms were washed each with 1 ml of distilled water for injection, dissolved in a buffer of pH 3 and analyzed by gel permeation chromatography. It was revealed that these water-insolubilized forms contained IL-2 and human serum albumin coexisting therein.

### Example 2

Lyophilized human serum albumin (10 mg) was added to an IL-2 solution (about 1 mg/ml, pH 5.0) in a 10-ml glass-made pointed tube. The mixture was shaken on a vortex mixer for 3 minutes, whereby IL-2 was water-insolubilized. The water-insolubilized form was collected by centrifugation at 3,500 rpm for 10 minutes and then redispersed by adding 1 ml of water. Centrifugation of the dispersion under the same conditions gave water-insolubilized IL-2.

To the thus-obtained water-insolubilized form was added 2 ml of 0.025 M glycine buffer (pH 3.5) for dissolution of said product. The potency of the resultant aqueous solution of IL-2 was determined, with an aqueous IL-2 solution (2 ml) containing 1 ml of the same IL-2 solution as used above as the starting material, 10 mg of human serum albumin and 1 ml of 0.025 M glycine buffer (pH 3.5) being used as a reference control. The results were: 12,500 U/ml for the aqueous solution derived from the water-insolubilized IL-2 form and 17,300 U/ml for the control aqueous solution. Therefore, when the yield (78%) of the water-insolubilized IL-2 is taken into consideration, the loss in IL-2 potency due to insolubilization can be judged to be little.

### Example 3

A 3-ml portion of an IL-2 stock solution (about 1 mg/ml, pH 5.0) and 30 mg of human serum albumin were placed in a 30-ml glass-made pointed tube, and the mixture was shaken on a vortex mixer for 5 minutes to give a water-insolubilized IL-2 with human serum albumin coexisting therein.

The water-insolubilized form was separated from the supernatant by centrifugation, then lyophilized and passed through a 350-mesh screen, and 2.4 mg of the thus-obtained product was suspended in 1 ml of physiological saline. The suspension was administered to a rabbit by intramucular injection into the femur. Blood samples were taken from the auricular vein 4 hours, 1 day and 4 days after administration and the serum IL-2 concentrations were determined by enzyme immunoassay using the sandwich technique with goat anti-recombinant IL-2 antibody as the first antibody and peroxidase-coupled rabbit anti-recombinant IL-2 antibody as the second antibody. The serum IL-2 concentration per milliliter was 44 ng after 4 hours, 1.1 ng after 1 day and 0.4 ng after 4 days. In vivo solubilization of the water-insolubilized IL-2 and sustained release of IL-2 were thus confirmed.

### Example 4

A 10-ml portion of an IL-2 stock solution (about 1 mg/ml, pH 5.0) was placed in a 30-ml glass-made pointed tube, and 0.5 ml of an aqueous human serum albumin solution having a concentration of 200 mg/ml was added. The aqueous IL-2 solution in said tube was shaken on a vortex mixer for 10 minutes to give a water-insolubilized IL-2 with human serum albumin coexisting therein.

The water-insolubilized form containing suspension was centrifuged at 3,000 rpm for 10 minutes, the supernatant was removed and, for washing the water-insolubilized form, 10 ml of distilled water was added for resuspending the water-insolubilized form therein. The suspension was again centrifuged at 3,000 rpm for 10 minutes, and the supernatant was removed. The water-insolubilized form containing dense slurry was lyophilyzed in the conventional manner, and the lyophilizate was passed through a 350-mesh screen. A 6.4-mg portion of the sieved, water-insolubilized IL-2 powder was dispersed uniformly in a solution of 0.5 g of polylactic acid-polyglycolic acid copolymer (PLGA) in 0.75 ml of methylene chloride using a vortex mixer. The dispersion was slowly added to 25 ml of 0.5% aqueous solution of polyvinyl alcohol while emulsification was effected by means of an ultrahigh velocity homogenizer (Kinematica, Germany). Thus was obtained an O/W emulsion with PLGA capsules dispersed in the aqueous phase. This O/W emulsion was stirred by means of a propeller stirrer for causing evaporation in water of the methylene chloride from the microcapsules. The resultant O/W emulsion was centrifuged, the supernatant was removed, and the microcapsules were redispersed in 1 ml of an aqueous solution containing 100 mg of mannitol. Lyophilization of the dispersion gave 387 mg of a mixture powder composed of the microcapsules and mannitol.

A portion of this mixture powder was dispersed in 1 ml of an aqueous solution containing 1 mg of polysorbate 80 and 50 mg of mannitol so that the dose as IL-2 amounted to 180 mcg. The dispersion was injected into the femur of a rabbit. Blood samples were taken from the auricular vein 1 day, 3 days, 14 days and 20 days after administration and the serum IL-2 concentrations were determined by enzyme immunoassay by the manner of Example 3. The serum IL-2 concentration per milliliter was 1.1 ng after 1 day, 1.0 ng after 3 days, 1.7 ng after 14 days and 0.6 ng after 20 days. Sustained release of IL-2 from the microcapsules was thus confirmed.

### Example 5

An IL-2 stock solution (about 1 mg/ml, pH 5.0), an IFN-α stock solution (about 1 mg/ml, pH 5.0) and an IFN-γ stock solution (about 2 mg/ml, pH 6.0) were each distributed in 1-ml portions into two vials and lyophilized. To the protein in each of the total of 6 vials for the three kinds of lyophilizate, there was added 5 mg of sodium alginate. Upon addition of 0.3 ml of water, a water-insolubilized IL-2, a water-insolubilized IFN-α and a water-insolubilized IFN-γ, each with sodium alginate coexisting therein. The suspension of each water-insolubilized form (one vial per product species) was lyophilized and the lyophilizate was sieved through a 100-mesh screen to give a lyophilized, powdery, water-insolubilized IL-2, IFN-α or IFN-γ. To each of the water-insolubilized form suspensions in the remaining vials, there was added 0.1 ml of an aqueous solution containing 5 mg of calcium chloride, whereby the excess sodium alginate was gelated. The gel was lyophilized and the lyophilizate was sieved through a 100-meshed screen to give a lyophilized, powdery alginate gel containing the water-insolubilized IL-2, IFN-α or IFN-γ.

### Example 6

Sodium alginate (50 mg) was added to and dissolved in 5 ml of an IL-2 stock solution (concentration about 1 mg/ml, pH 5.0) in a 30-ml glass-made pointed tube. The aqueous IL-2 solution in said tube was shaken on a vortex mixer for 10 minutes, whereby an IL-2-containing, water-insolubilized form was formed, with sodium alginate coexisting therein.

This water-insolubilized form suspension was centrifuged, the supernatant was removed, and the water-insolubiliezd form thus obtained was washed with distilled water and then lyophilized. The lyophilizate was passed through a 350-mesh sieve. A 1.5-mg portion of the sieved powder was dispersed uniformly in a solution containing 0.5 g of PLGA by the manner of Example 4, and the dispersion was processed in the manner of Example 4 to give 363 mg of a mixture powder composed of microcapsules and mannitol (containing 100 mg of mannitol). The whole quantity of this mixture powder was dispersed in 1ml of an aqueous solution containing 1 mg of polysorbate 80 and 50 mg of mannitol and the dispersion was injected intramucularly into the femur of a rabbit. Blood samples were taken from the auricular vein 1 day, 3 days, 7 days, 14 days, and 20 days after administration and the serum IL-2 concentrations were determined by enzyme immunoassay by the manner of Example 3. The IL-2 concentration per milliliter of serum was 0.3 ng after 1 day, 0.5 ng after 3 days, 0.4 ng after 7 days, 0.5 ng after 14 days and 0.2 ng after 20 days. Thus was confirmed sustained release of IL-2 from the microcapsules.

### Example 7

To a specified amount of an IL-2 stock solution (concentration about 1 mg/ml, pH 5.0), an IFN-α stock solution (concentration about 1 mg/ml, pH 5.0) or an IFN-γ stock solution (concentration about 2 mg/ml, pH 6.0), there was added lyophilized human serum albumin (HSA) to a concentration specified in Table 1, followed by shaking, which gave a water-insolubilized form.

The vessel used was a 10-ml glass-made pointed tube and the shaking was performed on a vortex mixer for 3 minutes. The water-insolubilized precipitate was collected by centrifugation (3,000 rpm, 10 minutes). After removal of the supernatant, 1 ml of 0.04 M phosphate buffer (pH 7.4) was added to the sediment for solubility evaluation. The results obtained are shown in Table 1.

**Table 1**

| Protein stock solution & volume | Amount of HSA added | Water-Insolubilized form | Solubility of water insolubilized form (pH 7.4) |
|---|---|---|---|
| IL-2, 0.5 ml | 5 mg | Formed | Insoluble |
| IFN-α, 0.5 ml | 5 mg | Formed | Insoluble |
| IFN-γ, 0.5 ml | 5 mg | Formed | Insoluble |
| IL-2, 1 ml | 200 mg | Formed | Insoluble |
| IFN-α, 1 ml | 200 mg | Formed | Insoluble |
| IFN-γ, 1 ml | 200 mg | Formed | Insoluble |

As is evident from Table 1, the water-insolubilized form formed were insoluble in 1 ml of the buffer at pH 7.4 in all the cases.

### Comparative Example 1

Sodium chloride (18 mg) was added to a portion of an IL-2 stock solution (about 1 mg/ml, pH 5.0) as placed in a 10-ml glass-made pointed tube, and the mixture was shaken on a vortex mixer for 3 minutes, whereby the IL-2 was insolubilized. Then, 1 ml of 0.1 M glycine buffer (pH 3.0) was added to the suspension containing the water-insolubilized IL-2, whereby the water-insolubilized IL-2 was dissolved. The IL-2 solution obtained (sample A) was assayed for potency, using, as a reference control, an IL-2 solution (sample B) having the same composition but not treated for IL-2 insolubilization. The results obtained are shown in Table 2. For the IL-2 potency assay, the proliferation of mouse natural killer cells which is dependent on the quantity of IL-2 was used as an index.

**Table 2**

| Potency of water-insolubilized IL-2 | | | |
|---|---|---|---|
| Sample | Composition (Per 2 ml) | Insolubilization of IL-2 | Potency/ml |
| A | IL-2 1 ml | Yes | 10,800 units/ml |
| | NaCl 18 mg | | |
| | Buffer (pH 3) 1 mg | | |
| B | IL-2 1 ml | No | 19,200 units/ml |
| | NaCl 18 mg | | |
| | Buffer (pH 3) 1 mg | | |

It was thus revealed that when sodium chloride is used, the water-insolubilized form shows a reduced IL-2 potency of below 60%.

### Example 8

The whole quantity of the water-insolubilized IL-2-containing dense slurry obtained by the method described in Example 4 was added to 10.0 g of soybean oil and uniformly dispersed therein using an ultrahigh velocity homogenizer (Kinematica) to give a W/O emulsion. The soybean oil was then dispersed in 100 ml of a 2.5% (w/w) aqueous solution of glycerol in a homogenizer with 1.2 g of yolk lecithin as the emulsifier. The thus-obtained W/O/W emulsion was distributed in 2-ml portions into vials under aseptic conditions, and each vial was fitted with a rubber stopper and a cap, followed by clinching. Thus a sustained release preparation for intramuscular injection of IL-2 was obtained.

### Example 9

A 5-mg portion of the lyophilized, powdery, water-insolubilized form obtained by the method described in Example 4 was uniformly dispersed in soybean oil using an ultrahigh velocity homogenizer, and this soybean oil was dispersed uniformly in 100 ml of a 2.5% (w/w) aqueous solution of glycerol by means of a homogenizer-mixer, with yokl lecithin used as the emulsifier, to give an O/W emulsion. This emulsion was distributed in 2-ml portions into vials under aseptic conditions. Each vial was fitted with a rubber stopper and a cap, followed by clinching. Thus a sustained release preparation for intramuscular injection of IL-2 was obtained.

### Example 10

The lyophilized water-insolubilized form obtained by the method described in Example 4 was passed through a 350-mesh screen and a 5-mg portion thereof was dispersed uniformly in 2 ml of an aqueous solution containing 200 mg of sodium alginate, and the dispersion was slowly added to 2 ml of an aqueous solution containing 100 mg of CaCl₂, whereby gelation of alginate was caused. The gel was lyophilized and the lyophilizate was passed through a 100-mesh screen to give a water-insolubilized IL-2-containing alginate gel powder. This alginate gel powder was distributed in 50-mg portions into vials under aseptic conditions. Each vial was fitted with a rubber stopper and a cap and then clinched. Thus a sustained release preparation for injection of IL-2 was obtained. This injectable preparation is suspended in 1 ml of physiological saline containing 2 mg of polysorbate 80 and administered intramuscularly to humans.

### Example 11

The lyophilized, water-insolubilized IL-2 obtained by the method described in Example 4 was passed through a 350-mesh screen and a 5-mg portion thereof was dispersed uniformly in 1 ml of an aqueous solution containing 200 mg of human serum albumin and 50 mg of reduced-form glutathione. Gelation of human serum albumin by reduced-form glutathione was caused by storing the aqueous solution at 40°C for 15 hours. Then, the resultant gel was lyophilizd, and the lyophilizate was passed through a 100-mesh screen to give a water-insolubilized IL-2-containing albumin gel powder. A 50-mg portion of this albumin gel powder was placed in a vial together with 9 mg of sodium chloride, and the vial was fitted with a rubber stopper and a cap and then clinched to give a sustained release IL-2 preparation for injection. This injectable preparation is suspended in 1 ml of distilled water for injection which contains 2 mg of polysorbate 80, for administration to a human by intramuscular injection.

### Example 12

A water-insolubilized IL-2-containing albumin gel powder was obtained by proceeding in the manner of Example 11 except that 40 mg of L-cysteine hydrochloride was used in lieu of 50 mg reduced-form glutathione, and then a sustained release preparation for injection was obtained using the albumin gel powder by the manner of Example 11. This injectable preparation is suspended in 1 ml of distilled water for injection containing 2 mg of polysorbate 80 for intramuscular administration to a human.

### Example 13

The lyophilized, powdery, water-insolubilized IL-2 obtained by the method described in Example 4 was passed through a 350-mesh screen and a 5-mg portion thereof was dispersed uniformly in 1 ml of an aqueous solution containing 200 mg of human serum albumin and then dissolved by addition of 20 mg of reduced-form glutathione. The aqueous solution was slowly added to 5 ml of ethanol while emulsification was effected by means of an ultrahigh velocity homogenizer (Kinematica). Thus a dispersion of microcapsules of human serum albumin was obtained. This dispersion was stored at 40°C for 15 hours for causing gelation of the human serum albumin by the coexisting reduced-form glutathione. The microcapsules resulting from the gelation were separated from the supernatant by centrifugation and then lyophilized to give a powder comprising water-insolublized IL-2-containing albumin microcapsules. A 30-mg portion of this albumin microcapsule powder was placed in a vial together with 50 mg of mannitol under aseptic conditions and the vial was fitted with a rubber stopper and a cap and then clinched to give a sustained release IL-2 preparation for injection. This injectable preparation is suspended in 1 ml of distilled water for injection containing 2 mg of polysorbate 80 for administration to a human by intramuscular injection.

### Example 14

A water-insolubilized IL-2-containing albumin microcapsule powder was obtained by proceeding in the manner of Example 13 except that 20 mg of L-cysteine hydrochloride was used in lieu of 20 mg of glutathione. A 30-mg portion of this albumin microcapsule powder was placed in a vial together with 30 mg of sodium chloride under aseptic conditions and the vial was fitted with a rubber stopper and a cap and then clinched to give a sustained release IL-2 preparation for injection. This injectable preparation is suspended in 1 ml of distilled water for injection containing 2 mg of polysorbate 80 for intramuscular injection to a human.

### Example 15

A 5-mg portion of the lyophilized, powdery, water-insolubilized IL-2 produced by the method described in Example 5 is used and the procedure of Example 4 is followed to give a mixture powder composed of mannitol and microcapsules containing a lyophilized, powdery, water-insolubilized IL-2. Then, 50 mg of this mixture powder is placed in a vial under aseptic conditions and the vial is fitted with a rubber stopper and a cap and then clinched to give a sustained release IL-2 preparation for injection. This injectable preparation is dispersed in 1 ml of an aqueous solution containing 2 mg of polysorbate 80 and 50 mg of mannitol for intramuscular administration to a human.

### Example 16

Using 5 mg of the lyophilized, water-insolubilized IL-2-containing alginate gel produced by the method described in Example 5 and proceeding by the manner of Example 15, there is obtained a sustained release IL-2 preparation for injection.

### Example 17

A mixture powder composed of mannitol and microcapsules containing a water-insolubilized IFN-α is obtained by the method described in Example 4 using 5 mg of a lyophilized, powdery, water-insolubilized IFN-α prepared by the manner of Example 4 using 10 ml of an IFN-α stock solution (concentration about 1 mg/ml, pH 5.0) in lieu of the IL-2 stock solution. Then, this mixture powder is distributed in 50-mg portions into vials. Each vial is fitted with a rubber stopper and a cap and then clinched. Thus a sustained release IFN-α preparation for injection is obtained. It is administered by the manner described in Example 15.

### Example 18

A sustained release IFN-α preparation for injection is obtained in the manner of Example 15 using 5 mg of the lyophilized, powdery, water-insolubilized IFN-α produced by the method described in Example 5.

### Example 19

A sustained release IFN-α preparation for injection is obtained by the manner of Example 15 using 5 mg of the lyophilized, water-insolubilized IFN-α-containing alginate gel powder produced by the method described in Example 5.

### Example 20

Using 5 mg of a lyophilized, water-insolubilized IFN-γ powder obtained in the manner described in Example 4 using 10 ml of an IFN-γ stock solution (concentration: about 2 mg/ml; pH 6.0) in lieu of 10 ml of the IL-2 stock solution, the procedure of Example 4 is followed to give a mixture powder composed of mannitol and microcapsules containing the water-insolubilized IFN-γ. This mixture powder is then distributed in 50-mg portions into vials. Each vial is then fitted with a rubber stopper and a cap, followed by clinching. Thus a sustained release IFN-γ preparation for injection is obtained. The method of administration is as described in Example 15.

### Example 21

A sustained release IFN-γ preparation for injection is obtained by the manner of Example 15 using 5 mg of the lyophilized, water-insolubilized IFN-γ powder produced by the method described in Example 5.

### Example 22

A sustained release IFN-γ preparation for injection is obtained by the manner of Example 15 using 5 mg of the lyophilized, water-insolubilized IFN-γ-containing alginate gel powder produced by the method described in Example 5.

### Example 23

A one milliliter portion of an IL-2 stock solution (concentration about 1 mg/ml, pH 5.0) and 100 mg of human serum albumin were placed in 10 ml glass-made pointed tube. After dissolution of the human serum albumin, 4 ml of methanol were added to the solution, whereby water-insolubilized IL-2 was formed.

The thus produced water-insolubilized IL-2 was recovered and dried. 40 mg of the dried sample in phosphate buffer saline was administered subcutaneously to rat. After 3 hours, one day and 3 days, bloods were collected from caudal vein, and the concentrations in serum were determined by enzyme immunoassay using the sandwich technique with goat anti-recombinant IL-2 antibody as the first antibody and peroxidase-coupled rabbit anti-recombinant IL-2 antibody as the second antibody. The serum IL-2 concentration per milliliter was 120 ng after 3 hours, 11.8 ng after one day and 1.11 ng after 3 days. In vivo solubilization of the water-insolubilized IL-2 and sustained release of IL-2 were thus confirmed.

### Example 24

A ten milliliter portion of an IL-2 stock solution (concentration about 1 mg/ml, pH 5.0) and 250 mg of human serum albumin were placed in 30 ml glass-made pointed tube. After dissolution of the human serum albumin, 20 ml of acetone were added to the solution, whereby water-insolubilized IL-2 was formed.

The thus produced water-insolubilized form-containing suspension was centrifuged at 3000 rpm for 10 minutes, and the supernatant was removed. The obtained water-insolubilized form was dried and powdered by mortar.

The thus obtained powder was dispersed uniformly in a solution of 4.5 g of polylactic acid-polyglycolic acid copolymer (PLGA) in 5.6 ml of methylene chloride using a vortex mixer. The dispersion was slowly added to 1000 ml of 0.5% polyvinyl alcohol while emulsification was effected by means of ultrahigh velocity homogenizer, whereby an O/W emulsion with PLGA capsules dispersed in the aqueous phase. This O/W emulsion was stirred by means of a propeller stirrer for causing evaporation in water of the methylene chloride from the microcapsules. The resultant O/W emulsion was centrifuged, the supernatant was removed, and the microcapsules were dispersed in 10 ml of an aqueous solution containing 100 mg of mannitol and lyophilized. A portion of this mixture powder in phosphate buffer saline was subcutaneously administered to rat so that the dose is 250 mcg as IL-2. Blood samples were collected from caudal vein 1 day after, 3 days after, 7 days after, and the IL-2 concentration in serum was measured by enzyme immunoassay by the manner of Example 3. The serum IL-2 concentration per milliliter was 1.26 ng after 1 day, 0.321 ng after 3 days, 0.425 ng after 7 days. Sustained release of IL-2 from the microcapsules was thus confirmed.

## Claims (Claims for the following Contracting State(s): AT, BE, CH, DE, FR, GB, IT, LI, LU, NL, SE)

1. A physiological activity-retaining and water-insolubilized form of a cytokine which is obtained by causing:
i) a physiologically adaptable protein selected from human serum albumin, human serum globulin, fibrinogen, collagen and casein; or
ii) a physiologically adaptable high molecular organic acid having a molecular weight of not less than 5000 selected from alginic acid, hyaluronic acid, chondroitinsulfuric acid, pectic acid, pectinic acid, heparinic acid, polyacrylic acid, pectin and carrageenan, or a salt thereof,
to act through physical stimulation on a cytokine said physical stimulation being shaking or stirring.

2. A water-insolubilized form of a cytokine as claimed in Claim 1, wherein the cytokine is a lymphokine.

3. A water-insolubilized form of a cytokine as claimed in Claim 2, wherein the lymphokine is interleukin-2.

4. A water-insolubilized form of a cytokine as claimed in Claim 2, wherein the lymphokine is interferon-α.

5. A water-insolubilized form of a cytokine as claimed in Claim 2, wherein the lymphokine is interferon γ.

6. A composition for injection which comprises a physiological activity-retaining and water-insolubilized form of a cytokine which is obtained by causing:
i) a physiologically adaptable protein selected from human serum albumin, human serum globulin, fibrinogen, collagen and casein; or
ii) a physiologically adaptable high molecular organic acid having a molecular weight of not less than 5000 selected from alginic acid, hyaluronic acid, chondroitinsulfuric acid, pectic acid, pectinic acid, heparinic acid, polyacrylic acid, pectin and carrageenan, or a salt thereof,
to act through physical stimulation on a cytokine said physical stimulation being shaking or stirring.

7. A composition as claimed in Claim 6, wherein the cytokine is a lymphokine.

8. A composition as claimed in Claim 7, wherein the lymphokine is interleukin-2.

9. A composition as claimed in Claim 7, wherein the lymphokine is interferon-α.

10. A composition as claimed in Claim 7, wherein the lymphokine is interferon-γ.

11. A method of producing a physiological activity retaining and water-insolubilized form of a cytokine, which comprises subjecting the cytokine to physical stimulation said physical stimulation being shaking or stirring in the presence of a physiologically adaptable protein selected from human serum albumin, human serum globulin, fibrinogen, collagen and casein or a physiologically adaptable high molecular organic acid having a molecular weight of not less than 5000 selected from alginic acid, hyaluronic acid, chondroitinsulfuric acid, pectic acid, pectinic acid, heparinic acid, polyacrylic acid, pectin and carrageenan, or a salt thereof, in an aqueous medium, whereby said protein or said organic acid acts on said cytokine to convert the cytokine to a water-insolubilized form.

12. A method as claimed in Claim 11, wherein said physiologically adaptable high molecular organic acid having a molecular weight of not less than 5000 or a salt thereof is caused to act on a cytokine in water.

13. A method as claimed in Claim 11, wherein said physiologically adaptable protein is caused to act on a cytokine in the presence of a water-soluble organic solvent.

14. A method as claimed in Claim 11, wherein the cytokine is a lymphokine.

15. A method as claimed in Claim 14, wherein the lymphokine is interleukin-2.

16. A method as claimed in Claim 14, wherein the lymphokine is interferon-α.

17. A method as claimed in Claim 14, wherein the lymphokine is interferon-γ.

## Claims (Claims for the following Contracting State(s): ES, GR)

1. A method of producing a physiological activity-retaining and water-insolubilized form of a cytokine, which comprises subjecting the cytokine to physical stimulation said physical stimulation being shaking or stirring in the presence of a physiologically adaptable protein selected from human serum albumin, human serum globulin, fibrinogen, collagen and casein or a physiologically adaptable high molecular organic acid having a molecular weight of not less than 5000 selected from alginic acid, hyaluronic acid, chondroitinsulfuric acid, pectic acid, pectinic acid, heparinic acid, polyacrylic acid, pectin and carrageenan, or a salt thereof, in an aqueous medium, whereby said protein or said organic acid acts, on said cytokine to convert the cytokine to a water-insolubilized form.

2. A method as claimed in Claim 1, wherein said physiologically adaptable high molecular organic acid having a molecular weight of not less than 5000 or a salt thereof is caused to act on a cytokine in water.

3. A method as claimed in Claim 1, wherein said physiologically adaptable protein is caused to act on a cytokine in the presence of a water-soluble organic solvent.

4. A method as claimed in Claim 1, wherein the cytokine is water-soluble.

5. A method as claimed in Claim 1, wherein the cytokine is a lymphokine.

6. A method as claimed in Claim 5, wherein the lymphokine is interleukin-2.

7. A method as claimed in Claim 5, wherein the lymphokine is an interferon.

8. A method as claimed in Claim 7, wherein said interferon is interferon-α.

9. A method as claimed in Claim 7, wherein said interferon is interferon - γ.

## Patentansprüche (Patentansprüche für folgende(n) Vertragsstaat(en): AT, BE, CH, DE, FR, GB, IT, LI, LU, NL, SE)

1. Die physiologische Aktivität erhaltende und wasserunlöslich gemachte Form eines Cytokins, die dadurch gewonnen wird, daß man
i) ein physiologisch anpassungsfähiges Protein, das aus humanem Serumalbumin, humanem Serumglobulin, Fibrinogen, Collagen und Casein ausgewählt ist; oder
ii) eine physiologisch anpassungsfähige hochmolekulare organische Säure mit einem Molekulargewicht nicht unter 5000, die aus Alginsäure, Hyaluronsäure, Chondroitinschwefelsäure, Pectinsäure, pectiniger Säure, Heparinsäure, Polyacrylsäure, Pectin und Carrageenan oder einem Salz davon ausgewählt ist,
mittels physikalischer Stimulation auf ein Cytokin einwirken läßt, wobei die physikalische Stimulation aus Schütteln oder Rühren besteht.

2. Wasserunlöslich gemachte Form eines Cytokins nach Anspruch 1, worin das Cytokin ein Lymphokin ist.

3. Wasserunlöslich gemachte Form eines Cytokins nach Anspruch 2, worin das Lymphokin Interleukin-2 ist.

4. Wasserunlöslich gemachte Form eines Cytokins nach Anspruch 2, worin das Lymphokin Interferon-α ist.

5. Wasserunlöslich gemachte Form eines Cytokins nach Anspruch 2, worin das Lymphokin Interferon-γ ist.

6. Zusammensetzung zur Injektion, umfassend eine die physiologische Aktivität erhaltende und wasserunlöslich gemachte Form eines Cytokins, die dadurch gewonnen wird, daß man
i) ein physiologisch anpassungsfähiges Protein, das aus humanem Serumalbumin, humanem Serumglobulin, Fibrinogen, Collagen und Casein ausgewählt ist; oder
ii) eine physiologisch anpassungsfähige hochmolekulare organische Säure mit einem Molekulargewicht nicht unter 5000, die aus Alginsäure, Hyaluronsäure, Chondroitinschwefelsäure, Pectinsäure, pectiniger Säure, Heparinsäure, Polyacrylsäure, Pectin und Carrageenan oder einem Salz davon ausgewählt ist,
mittels physikalischer Stimulation auf ein Cytokin einwirken läßt, wobei die physikalische Stimulation aus Schütteln oder Rühren besteht.

7. Zusammensetzung nach Anspruch 6, worin das Cytokin ein Lymphokin ist.

8. Zusammensetzung nach Anspruch 7, worin das Lymphokin Interleukin-2 ist.

9. Zusammensetzung nach Anspruch 7, worin das Lymphokin Interferon-α ist.

10. Zusammensetzung nach Anspruch 7, worin das Lymphokin Interferon-γ ist.

11. Verfahren zur Herstellung einer die physiologische Aktivität erhaltende und wasserunlöslich gemachte Form eines Cytokins, umfassend das Einwirkenlassen einer physikalischen Stimulation auf das Cytokin, wobei die physikalische Stimulation aus Schütteln oder Rühren in Gegenwart eines physiologisch anpassungsfähigen Proteins, das aus humanem Serumalbumin, humanem Serumglobulin, Fibrinogen, Collagen und Casein ausgewählt ist oder einer physiologisch anpassungsfähigen hochmolekularen organischen Säure mit einem Molekulargewicht nicht unter 5000, die aus Alginsäure, Hyaluronsäure, Chondroitinschwefelsäure, Pectinsäure, pectiniger Säure Heparinsäure, Polyacrylsäure, Pectin und Carrageenan oder einem Salz davon ausgewählt ist, in einem wäßrigen Medium besteht, wobei das Protein oder die organische Säure auf das Cytokin einwirkt, um das Cytokin in eine wasserunlöslich gemachte Form zu überführen.

12. Verfahren nach Anspruch 11, worin man die physiologisch anpassungsfähige hochmolekulare organische Säure, mit einem Molekulargewicht nicht unter 5000, oder ein Salz davon auf ein Cytokin in Wasser einwirkenläßt.

13. Verfahren nach Anspruch 11, worin man das physiologisch anpassungsfähige Protein auf ein Cytokin in Gegenwart eines wasserlöslichen organischen Lösungsmittels einwirken läßt.

14. Verfahren nach Anspruch 11, worin das Cytokin ein Lymphokin ist.

15. Verfahren nach Anspruch 14, worin das Lymphokin Interleukin-2 ist.

16. Verfahren nach Anspruch 14, worin das Lymphokin Interferon-α ist.

17. Verfahren nach Anspruch 14, worin das Lymphokin Interferon-γ ist.

## Patentansprüche (Patentansprüche für folgende(n) Vertragsstaat(en): ES, GR)

1. Verfahren zur Herstellung einer die physiologische Aktivität erhaltende und wasserunlöslich gemachte Form eines Cytokins, umfassend das Einwirkenlassen einer physikalischen Stimulation auf das Cytokin, wobei die physikalische Stimulation aus Schütteln oder Rühren in Gegenwart eines physiologisch anpassungsfähigen Proteins, das aus humanem Serumalbumin, humanem Serumglobulin, Fibrinogen, Collagen und Casein ausgewählt ist oder einer physiologisch anpassungsfähiges hochmolekularen organischen Säure mit einem Molekulargewicht nicht unter 5000, die aus Alginsäure, Hyaluronsäure, Chondroitinschwefelsäure, Pectinsäure, pectiniger Säure Heparinsäure, Polyacrylsäure, Pectin und Carrageenan oder einem Salz davon ausgewählt ist, in einem wäßrigen Medium besteht, wobei das Protein oder die organische Säure auf das Cytokin einwirkt, um das Cytokin in eine wasserunlöslich gemachte Form zu überführen.

2. Verfahren nach Anspruch 1, worin man die physiologisch anpassungsfähige hochmolekulare organische Säure, mit einem Molekulargewicht nicht unter 5000, oder ein Salz davon auf ein Cytokin in Wasser einwirken läßt.

3. Verfahren nach Anspruch 1, worin man das physiologisch anpassungsfähige Protein auf ein Cytokin in Gegenwart eines wasserlöslichen organischen Lösungsmittel einwirken läßt.

4. Verfahren nach Anspruch 1, worin das Cytokin wasserlöslich ist.

5. Verfahren nach Anspruch 1, worin das Cytokin ein Lymphokin ist.

6. Verfahren nach Anspruch 5, worin das Lymphokin Interleukin-2 ist.

7. Verfahren nach Anspruch 5, worin das Lymphokin ein Interferon ist.

8. Verfahren nach Anspruch 7, worin das Interferon Interferon-α ist.

9. Verfahren nach Anspruch 7, worin das Interferon Interferon-γ ist.

## Revendications (Revendications pour l'(les) Etat(s) contractant(s) suivant(s): AT, BE, CH, DE, FR, GB, IT, LI, LU, NL, SE)

1. Forme de cytokine conservant son activité physiologique et rendue insoluble dans l'eau, obtenue en faisant agir
i) une protéine physiologiquement acceptable choisie parmi l'albumine sérique humaine, la globuline sérique humaine, le fibrinogène, le collagène et la caséine, ou
ii) un acide organique macromoléculaire, physiologiquement acceptable, d'un poids moléculaire non inférieur à 5000, choisi parmi l'acide alginique, l'acide hyaluronique, l'acide chondroïtinesulfurique, l'acide pectique, l'acide pectinique, l'acide héparinique, l'acide polyacrylique, la pectine et le carragheen ou un de leurs sels,
sur une cytokine, par stimulation physique, ladite stimulation physique consistant à secouer ou agiter.

2. Forme de cytokine rendue insoluble dans l'eau, selon la revendication 1, dans laquelle la cytokine est une lymphokine.

3. Forme de cytokine rendue insoluble dans l'eau, selon la revendication 2, dans laquelle la lymphokine est l'interleukine-2.

4. Forme de cytokine rendue insoluble dans l'eau, selon la revendication 2, dans laquelle la lymphokine est l'interféron-α.

5. Forme de cytokine rendue insoluble dans l'eau, selon la revendication 2, dans laquelle la lymphokine est l'interféron-γ.

6. Composition injectable, qui comprend une forme de cytokine conservant son activité physiologique et rendue insoluble dans l'eau, obtenue en faisant agir
i) une protéine physiologiquement acceptable choisie parmi l'albumine sérique humaine, la globuline sérique humaine, le fibrinogène, le collagène et la caséine, ou
ii) un acide organique macromoléculaire, physiologiquement acceptable, d'un poids moléculaire non inférieur à 5000, choisi parmi l'acide alginique, l'acide hyaluronique, l'acide chondroïtinesulfurique, l'acide pectique, l'acide pectinique, l'acide héparinique, l'acide polyacrylique, la pectine et le carragheen ou un de leurs sels,
sur une cytokine, par stimulation physique, ladite stimulation physique consistant à secouer ou agiter.

7. Composition selon la revendication 6, dans laquelle la cytokine est une lymphokine.

8. Composition selon la revendication 7, dans laquelle la lymphokine est l'interleukine-2.

9. Composition selon la revendication 7, dans laquelle la lymphokine est l'interféron-α.

10. Composition selon la revendication 7, dans laquelle la lymphokine est l'interféron-γ.

11. Procédé de préparation d'une forme de cytokine conservant son activité physiologique et rendue insoluble dans l'eau, selon lequel on soumet la cytokine à une stimulation physique, ladite stimulation physique consistant à secouer ou agiter, en présence d'une protéine physiologiquement acceptable choisie parmi l'albumine sérique humaine, la globuline sérique humaine, le fibrinogène, le collagène et la caséine, ou d'un acide organique macromoléculaire, physiologiquement acceptable, d'un poids moléculaire non inférieur à 5000, choisi parmi l'acide alginique, l'acide hyaluronique, l'acide chondroïtinesulfurique, l'acide pectique, l'acide pectinique, l'acide héparinique, l'acide polyacrylique, la pectine et le carragheen ou un de leurs sels, dans un milieu aqueux, ladite protéine ou ledit acide organique agissant sur ladite cytokine pour transformer la cytokine en une forme rendue insoluble dans l'eau.

12. Procédé selon la revendication 11, dans lequel on fait agir ledit acide organique macromoléculaire physiologiquement acceptable, d'un poids moléculaire non inférieur à 5000, ou un sel de celui-ci sur une cytokine dans l'eau.

13. Procédé selon la revendication 11, dans lequel on fait agir ladite protéine physiologiquement acceptable sur une cytokine en présence d'un solvant organique soluble dans l'eau.

14. Procédé selon la revendication 11, dans lequel la cytokine est une lymphokine.

15. Procédé selon la revendication 14, dans lequel la lymphokine est l'interleukine-2.

16. Procédé selon la revendication 14, dans lequel la lymphokine est l'interféron-α.

17. Procédé selon la revendication 14, dans lequel la lymphokine est l'interféron-γ.

## Revendications (Revendications pour l'(les) Etat(s) contractant(s) suivant(s): ES, GR)

1. Procédé de préparation d'une forme de cytokine conservant son activité physiologique et rendue insoluble dans l'eau, selon lequel on soumet la cytokine à une stimulation physique, ladite stimulation physique consistant à secouer ou agiter, en présence d'une protéine physiologiquement acceptable choisie parmi l'albumine sérique humaine, la globuline sérique humaine, le fibrinogène, le collagène et la caséine, ou d'un acide organique macromoléculaire, physiologiquement acceptable, d'un poids moléculaire non inférieur à 5000, choisi parmi l'acide alginique, l'acide hyaluronique, l'acide chondroïtinesulfurique, l'acide pectique, l'acide pectinique, l'acide héparinique, l'acide polyacrylique, la pectine et le carragheen ou un de leurs sels, dans un milieu aqueux, ladite protéine ou ledit acide organique agissant sur ladite cytokine pour transformer la cytokine en une forme rendue insoluble dans l'eau.

2. Procédé selon la revendication 1, dans lequel on fait agir ledit acide organique macromoléculaire physiologiquement acceptable, d'un poids moléculaire non inférieur à 5000, ou un sel de celui-ci sur une cytokine dans l'eau.

3. Procédé selon la revendication 11, dans lequel on fait agir ladite protéine physiologiquement acceptable sur une cytokine en présence d'un solvant organique soluble dans l'eau.

4. Procédé selon la revendication 1, dans lequel la cytokine est soluble dans l'eau.

5. Procédé selon la revendication 1, dans lequel la cytokine est une lymphokine.

6. Procédé selon la revendication 5, dans lequel la lymphokine est l'interleukine-2.

7. Procédé selon la revendication 5, dans lequel la lymphokine est un interféron.

8. Procédé selon la revendication 7, dans lequel ledit interféron est l'interféron-α.

9. Procédé selon la revendication 7, dans lequel ledit interféron est l'interféron-γ.
